(19) Europäisches Patentamt European Patent Office Office européen des brevets

(11) **EP 4 646 965 A1**

(12) **EUROPEAN PATENT APPLICATION**

(43) Date of publication:
**12.11.2025 Bulletin 2025/46**

(21) Application number: **25170943.2**

(22) Date of filing: **16.04.2025**

(51) International Patent Classification (IPC):
**A45D 44/00** $^{(2006.01)}$   **A47K 5/12** $^{(2006.01)}$
**A61M 35/00** $^{(2006.01)}$

(52) Cooperative Patent Classification (CPC):
**A45D 44/005; A61M 35/25;** A45D 2044/007;
A47K 5/1217

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.05.2024 IT 202400010444**

(71) Applicant: **Sihealth Photonics S.r.l.**
**57121 Livorno (IT)**

(72) Inventors:
• **SIMEONE, Emilio**
  **57121 LIVORNO (IT)**
• **MORELLI, Marco**
  **57121 LIVORNO (IT)**

(74) Representative: **Nesti, Antonio**
  **Praxi Intellectual Property S.p.A.**
  **Via Cristoforo Landino, 14**
  **50129 Firenze (IT)**

(54) **PROTECTIVE SUNSCREEN DISPENSING APPARATUS**

(57)    Protective sunscreen dispensing apparatus, including a dispenser (1) equipped with dispensing means (2) that can be operated to repeatedly dispense a dose of product (Pk) contained in the Tank (Sk), detection means (15) configured for the detection of unit dispensing made, selection means (7) configured to select a part (Cj) of the body to be protected; a wireless interface (3a) configured to receive from an electronic unit (16) the number (Ni) of dispensing detected and transmit it to a communication interface (3b) for use by a user of the device, a computer program (APP) stored and executable in the portable device (SP) which, if executed, calculates a quantity (Qjk) of product (Pk) necessary to obtain the desired protection of one or more selected parts (Cj) of the user's body and the number (Njk) of doses (D) to be dispensed to obtain the calculated quantity of product (Qjk), receives from the dispenser (1) the current number (Ni) of dispenses made, and calculates the number of remaining dispenses (Njk-Ni) still to be performed to reach the total calculated number (Njk) and makes it known to the user.

FIG.2

EP 4 646 965 A1

## Description

### Field of the Invention

The invention concerns an intelligent dispensing apparatus to allow one or more users to apply a correct and controlled amount of lotion or protective sunscreen to one or more parts of the body exposed to the sun.

### State of the art

It is well known that adequate protection from solar radiation is one of the measures, if not the most effective measure, to reduce the incidence of diseases, or even just skin damage caused by sun exposure.

For this purpose, sunscreen products, such as creams or lotions, are widely used on the market and characterized by a protection factor according to which the application of the lotion or cream must be carried out with a specific surface thickness (e.g. 2 mg/cm2 in the standard case according to the guidelines issued by the European Cosmetics Association COLIPA ®) in order to guarantee the indicated protection.

In reality, in fact, several clinical studies show on the one hand that users rarely apply the prescribed amount of product and on the other hand that the amount applied to the different parts of the body is not at all uniform, with the result that the protective power is very different from that known in laboratory conditions and typically very different for different parts of the body.

A first unresolved technical problem at the state of the art is therefore the possibility for the user to be able to dose, for each part of the body, a correct amount of product to be applied or at least to make known the quantity applied to know its actual protective power.

A system with one or more UV sensors coupled to a smartphone and configured to measure UV radiation has been known for US9551611 so that the smartphone, communicating with the sensors, receives the measurements of UV radiation and executes an algorithm through an application that indicates the cumulative UV exposure to the user based on the positions assumed and the time duration of the exposure. This document also describes a dispenser synchronized via Bluetooth with an application loaded on the user's smartphone and equipped with a switch that is activated when the plunger for dispensing sunscreen is pressed, signaling to the smartphone the event of use of the dispenser corresponding to an event of application of the cream.

With this solution, the smartphone monitors the events of use of the dispenser via the application, sending the user reminders for the use of the protective product and adjusts the recommended reapplication time, based on weather conditions, location and UV index.

This well-known system addresses the problem of providing the user with a tool to know how much radiation he has received and when the user has applied the cream on his body and when he will have to reapply it, tracking his behavior thanks to the recording of the use of the dispenser.

This known system does not consider in any way the quantity of product applied through the use of the dispenser and even less does it consider the photoprotective effectiveness of the applied product which derives from the thickness deriving from the quantity actually applied to the body.

The systems of a known type therefore have the disadvantage of not allowing an adequate preliminary preparation for a user who has to expose himself to solar radiation, guiding him in the correct application of the photoprotective cream on the various parts of the body with the consequence that it is in fact impossible to check and verify a posteriori the real protection factor obtained with the application of the product on the skin.

### Aim of the invention

The need is therefore felt of an apparatus that allows a user to precisely control the quantity of photoprotective product, cream or lotion, which must be applied to the different areas of the body in order to be sure of the amount of cream or lotion actually applied to each part of the body and which can thus provide the same user with reliable information on the protective power of the product applied, guiding it during application in order to obtain the desired protection and also providing the recommended exposure times for the different effects deriving from sun exposure (e.g. maximum exposure time to avoid the onset of erythema, minimum exposure time to obtain the daily dose of vitamin D).

### Summary of the invention

These aims have been achieved by an apparatus according to the attached claims including a supplier or dispenser that counts the number of dispensations of cream or lotion to be spread on one or more parts of the body, following a protocol set by a program or application run on a mobile device, such as a user's smartphone, which informs the user in advance on how many dispensations to apply to each part of the body according to a specific biometric model for each area of the body, for example based on weight and height and possibly of the clothes worn and which during application allows you to check that the amount dispensed to be applied to the skin is actually the correct one or in any case known to be able to estimate the actual photoprotective power of the applied cream.

Thanks to the apparatus of the invention it is possible, in fact, for a given chemical composition of the cream, to determine the mathematical model and the parameters that characterize it to evaluate the effective photoprotective factor of the product to be applied, determining the thickness to be obtained for each part of the body and making sure that the thickness of the product then actually applied by the user on each part of the body is determined in a reliable and repeatable way.

According to the invention, the dispenser is equipped with a communication interface with the application, for example a Bluetooth ® radio frequency interface, to send the number of dispensations made on which the algorithm of the application bases the calculation of the thickness of cream actually spread on each part of the body.

In an example of embodiment, the dispenser will be able to have several tanks separated by different degrees of protection for different parts of the body or for different people.

A first advantage is given by the fact that the device is able to provide the user with timely information about the amount of photoprotective product to be applied to each part of the body before exposure to the sun and about the safe or recommended exposure times for a given health effect (first of all the risk of erythema, but also photoaging damage or DNA damage) predicted according to the specific characteristics of sensitivity of the skin to solar radiation (e.g. phototype and minimum erythemogenic dose), and/or the expected solar radiation conditions and/or the reflective surfaces in which it is expected to be exposed (albedo).

A second advantage consists in the fact that thanks to the knowledge of the actual protective factor obtained from the controlled application of the photoprotective product depending on the thickness of the product applied, it is possible to provide the user with additional information for therapeutic effects or in any case for the benefit of the user's health, such as the calculation of the minimum exposure time to obtain the recommended dose of vitamin D that will be obtained thanks to exposure solar, if the user's clothing during the exposure will also be known in particular.

A further advantage is that the information provided by the apparatus can also be used both to accurately predict the different effects of the user's preparation for sun exposure (e.g. maximum exposure time corresponding to a minimum erythemogenic dose of the user (MED), minimum exposure time to obtain the recommended daily dose of vitamin D, ..) and to be able to monitor sun exposure (e.g. monitoring of the actual dose of UV radiation that reaches the skin in every part of the body due to the thickness and declared protective factor of the product applied).

An even further advantage is that the same device can be used as a "multi-user" to manage the protection of several people.

### List of drawings

These and other advantages will be better understood by any skilled person in the field, from the description that follows and from the attached drawings, given as a nonlimiting example, in which:

- Fig.1 shows an elevation view of a dispenser according to the invention;
- Fig.1a shows a magnified view from above of the dispenser in Fig.1;
- Fig.2 schematically shows an apparatus according to the invention;
- Fig.3 schematically shows an example of an electronic section of an apparatus according to the invention.

### Detailed Description

With reference to the attached drawings, an apparatus for the controlled dispensing of protective sunscreen according to the invention is now described.

The apparatus includes a dispenser 1 preferably composed of a container 10 of at least one photoprotective product, closed by a lid 9, preferably of the removable type, equipped in turn with dispensing means formed in the example illustrated by a dosing device 2 that can be operated repeatedly to release from container 10 a constant dose D of at least one product Pk contained in the dispenser and characterized by its own nominal protective factor FPk.

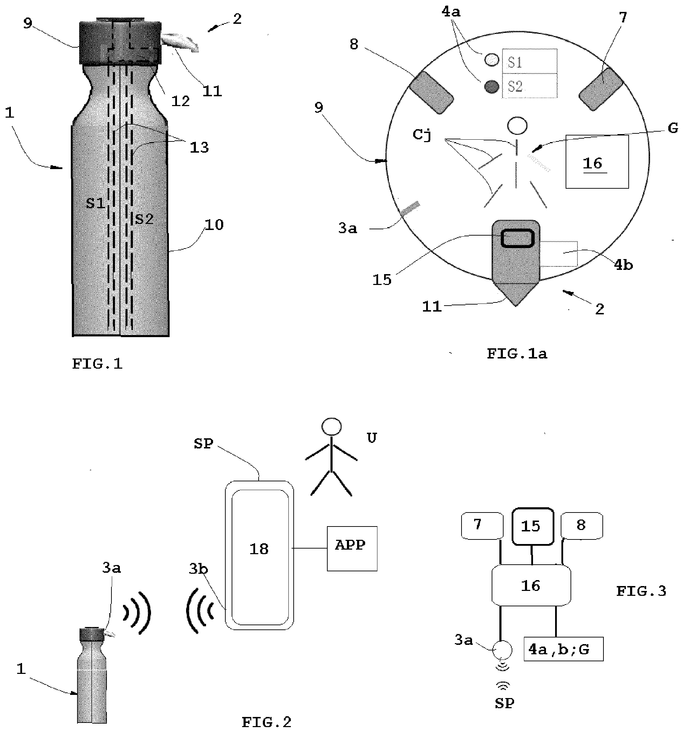

By way of example, figure 1 shows a dispenser according to the invention in which container 10 is divided into two tanks S1, S2 which can contain sunscreens or lotions P1, P2 with different protective factor FP1, FP2 and which can be dispensed through a spout 11 of dosing device 2 through a suction mechanism, for example, a manual mechanism of a type known in itself including a pump 12 that communicates at the top with the spout 11 to expel the product and at the bottom with the respective tank S1, S2 through the respective suction tubes 13 to suck the photoprotective product to be dispensed.

In operation, the dosing device 2 therefore allows a constant known quantity D of each product Pk to be extracted from the container for each i-th event of use of it.

In this way, the quantity extracted of cream or lotion from the Sk tank (in the case illustrated k = 1, 2) in use can be expressed as:

$$Qk = N \times D$$

where N is the number of individual uses of the dosing device and D is the unit dose amount of the product Pk extracted per single use of the dosing device.

According to the invention, dosing device 2 is equipped with a sensor 15 (e.g. a digital on-off switch) connected to an electronic unit 16 of the dispenser, housed in the case described in lid 9 and configured to count the number of dispensations performed and detected by sensor 15.

According to the diagram illustrated in fig.3, electronics 16 is also connected to a 3a communication interface, e.g. radio frequency, to transmit in real time to a user's SP terminal, e.g. a portable device/smartphone, the information of the extraction of each unit dose from the tank (possibly accompanied by an acoustic and/or light signal, e.g. an LED light).

The SP terminal is in turn equipped with a communication interface 3b capable of exchanging data with interface 3a and its own electronics capable of executing an APP application for processing the data coming from dosing device 2.

In preferred forms of implementation, the electronics 16 of dosing device 2 can also be connected to a selector 8, preferably an electronic selector, possibly remotely controlled from the SP terminal through the same communication interface 3a, in order to direct the suction of dosing device 2 from one of the available tanks, through which the user can connect the dispenser to the tank in which a known Pk product is contained (e.g. tank S1 with protection factor FP=30 and tank S2 with protection factor FP=50) to extract the desired quantity through a number N of unit doses D.

In this case, moreover, the tank on which the dosing device has been activated can optionally be indicated to the user by a light indicator 4a (for example a different coloured LED light for each tank) positioned on the lid 9 of the dispenser (fig.1a) while an identifier of the active tank, represented in fig.1a by the codes S1, S2, can be communicated to the APP of the SP terminal by the electronics 16 of the dispenser which will thus be able to evaluate the quantity extracted by the user from the tank on which the selector has directed the dosing device.

According to the invention, the SP handheld terminal APP program when run by the user calculates a Qjk amount of the product contained in the selected Sk tank which is required to achieve the desired protection i.e. the actual protection factor FPEjk for one or more selected parts Cj of the body.

The desired actual protection factor FPEjk will in turn have been previously evaluated by the APP program on the basis of the photoprotective factor FPk of the product contained in the Sk tank and by other factors accessible to the APP program and relevant for the photoprotection of the skin for the body part Cj (e.g. parameters associated with the user such as sensitivity to solar radiation, personal data, any pathologies etc..) and finally by external factors such as the current and expected UV radiation for the location in which the user is exposed and possibly the time of exposure of the user to the desired solar radiation.

Once the desired effective protection factor FPEjk has been calculated, the APP program will calculate the quantity Qjk of product to be applied based on the width of the skin surface Aj of the part of the body to be protected Cj and the thickness tjk of photoprotective product with nominal protection factor FPk necessary to obtain the desired FPEjk value.

In this regard, it should be remembered that the thickness t_standard for which the protection factor declared for the marketing of the photoprotective cream is obtained and for which laboratory tests have been carried out is set by the certification standard. For example, for the COLIPA standard the value of this certification thickness t_standard is 2 mg/cm2. In the event that the thickness tjk used for the calculation of Qjk is equal to 2 mg/cm2, the effective protective factor FPEjk will therefore be exactly the certified nominal FPk, otherwise it will be less or greater than it, for thicknesses lower or higher respectively. The actual protection factor FPEjk applied can be reliably calculated in different ways as a function of the thickness tjk taking into account the specificity of the composition of the cream, through reliable mathematical models available in the literature.

In particular, it is known, for example, that the value of the effective protection factor FPE as a function of thickness "t" typically follows a linear function for low protection factors FP according to the COLIPA standard (e.g. FP from 4 to 15) while it follows an exponential-logarithmic trend according to the classical law of extinction of radiation in absorbing media for higher protection factors (e.g. FP 30 - 50). Typically, we have:

$$FPE = a*t + b \qquad per\ FP < 15$$
$$FPE = C*exp\ (\ K*t) \qquad per\ FP > 15$$

where the coefficients a, b or C and K will be determined for a given photoprotective dermocosmetic product depending on the chemical composition and spectral response of the product itself.

The thickness tjk associated with the desired FPEjk value will then be obtained from value association tables or inverse formulas of these models or other more sophisticated ones available from literature or simulators available on-line.

The thickness tjk for a given section of the body Cj of area Aj in cm2 and for the quantity Qjk applied in mg of the product contained in the tank Sk, will assume an average value tjk on the part of the body Cj given by:

$$tjk = Qjk / Aj$$

so we have:

$$Qjk = tjk * Aj$$

In un esempio di funzionamento dell'apparato, Aj viene valutato dal programma APP dell'invenzione facendo uso di un modello matematico biometrico del corpo umano dell'utente allo scopo di calcolare la superficie corporea (Body Surface Area - BSA) secondo un algoritmo che valuta attraverso un calcolo biometrico la superficie della pelle del corpo dalla altezza e dal peso .

Some models known to obtain the surface area in BSA in m2, through the data of height A in cm and weight P in Kg are for example:

| Formula | Superficie corporea (in m$^2$) |
|---|---|
| Mosteller | $BSA = \sqrt{\dfrac{A \times P}{3600}}$ |
| DuBois | $BSA = (0,007184 \times A^{0,725} \times P^{0,425})$ |
| Haycock | $BSA = (0,024265 \times A^{0,3964} \times P^{0,5378})$ |
| Gehan e George | $BSA = (0,0235 \times A^{0,42246} \times P^{0,51456})$ |

In any case, regardless of the calculation model chosen, the invention APP program is configured to divide the human body into different parts or sectors Cj (e.g., Scalp, Face, Neck, Right Shoulder, Left Shoulder, Right Arm, Left Arm, Right Leg, Left Leg, Right, Left Shoulder, Chest, Back, Feet), and for each Cj sector of the body the respective area Aj is calculated by means of a biometric formula as a percentage of the total area BSA, i.e.:

$$Aj = \%j * BSA.$$

Biometric models for the calculation of this %j used in clinical practice (e.g. for burned areas) are available from the literature (e.g. Lund-Browder model).

In addition, it is envisaged that the calculation of the surface Aj actually exposed can be refined through an evaluation of the effect of skin coverage due to the clothes worn by the user (through, for example, an estimate with artificial intelligence from a photograph of the user or an interactive questionnaire with the user from a smartphone application).

Once the quantity of product Qjk to be dispensed has been obtained, the total number Njk of Djk doses to be dispensed and applied to the skin to obtain photoprotection of one or more Cj parts of the user's body for the Sk tank is obtained by dividing the total quantity Qjk by the unit dose D that can be dispensed by dispenser 2 i.e.:

$$Njk = Qjk / D.$$

In use, the user starts the APP program to control the application of a certain photoprotective product Pk contained in the dispenser in the Sk tank on one or more parts of the body.

Preferably the APP proposes to the user a procedure for the application of the product Pk and the tank Sk in which it is contained, starting from a given part of the body Cj, applying the doses provided for that part. Once this application has been completed, the APP suggests the user to start applying a new Pk product to the C(j+1) part, until the application is completed for all the C parts of the body. In order to guide the user, the tank Sk and the part of the human body Cj of interest to which the dose count is referring can be indicated by a graphic on the screen of the SP user terminal and/or on the lid 9 through the indicators 4a of the tank and a graphic diagram G that shows through the activation of a light indicator (e.g. LED). In addition, it may be provided that a specific light and acoustic signal of the container signals the end of the application of the cream or lotion to the given sector of the body so that the user prepares for the application of the next sector of the body C(j+1) and possibly by another Sk. tank.

When using the device, as the user dispenses a single dose of the dispenser for the part of the body Cj in question, this event is reported by dispenser 1 both to the SP user terminal and through the APP program it counts the doses to be dispensed and provides information on the number of doses still to be dispensed for that part of the body from the Sk. tank. For this part of the body Cj, after calculating the total number Njk of doses necessary for the protection of the selected body part, APP receives from dosing device 2 the number "i" of deliveries gradually made and based on the number of deliveries performed, returns to dosing device 2 the number of remaining deliveries still to be performed to reach the total number calculated Njk, which may be displayed on screen 18 of the terminal and/or on a display 4b of the dispenser in order to allow direct control by the user.

However, it is understood that the control of the achievement of the desired number of dispenses Njk may be carried out in other ways, for example, by means of acoustic or visual signals or by blocking the dispenser when the desired number of dispenses is reached.

Preferably, the electronics 16 of the dispenser can be connected to a selector 7, preferably an electronic selector possibly remotely controlled from the SP terminal via the same communication interface 3a, to select the part of the body Cj to be protected. Advantageously, the user in possession of the SP terminal will thus be guided during the application process by being able to control the actual delivery of the necessary amount of Qkj cream or the amount that will be extracted from the k-th tank to be applied by the user to one or more j-th part of the body.

In a further example of implementation, the user is not obliged to follow the number of individual doses of product to be applied and can at any time move to the next sector ahead of the number of applications of Njk doses, indicating it either through the interface of the SP intelligent terminal, or for convenience, through the selector 7 or through a STOP button optionally present on the container.

In this case, the App program will calculate and communicate to the user the effective protection factor FEPjk resulting from the number Njk of the individual doses delivered for the body part Cj and indicating the resulting exposure time for the different effects (e.g. maximum exposure time to avoid the erythemogenic dose and minimum exposure time to obtain the recommended daily dose of vitamin D).

In addition, the user's portable intelligent terminal, knowing the capacity of the container tank and the unitary dose of the dosing device, through the APP program will be able to calculate the current content starting from a signal event of the tank being filled, and to warn the user when it is necessary to refuel.

Through the APP program for each sector of the body Cj, the user can also be recommended a photoprotective product Pk contained in the Sk, with specific characteristics for the skin for that part of the body, also with an appropriate nominal protective factor FPk (e.g. based on its degree of exposure such as the shoulders, and/or its specific sensitivity to photoaging such as the face and scalp, specific hydration for the face, .) and on the basis of these data (surface Aj to be protected and protective factor of the applied product FPk), the quantity of product Qjk to be applied through dosing device 2 is calculated, as shown above.

Preferably, the APP program can also be configured to calculate parameters useful for determining the quantity of product to be dispensed, such as the user's phototype, sensitivity to solar radiation (e.g. Minimum Erythemogenic Dose - MED) and the solar radiation expected for the different parts of the body, and in this case it can also inform the user of the expected exposure times for each Cj sector of the body to be protected. In an example of use of particular interest, the device can also be used as a "multi-user" to manage the application of the photoprotective product for several people, either using a single terminal (e.g. managed by a mother of a family) or for users each equipped with their own terminal (e.g. a group of friends who share the same dispenser). In this case, once the product has been applied for a given person, from a new terminal you can connect again to the same container to manage the protection of a new person.

The invention's APP program can also be configured to implement a "memo" function for the reapplication of the cream according to relevant parameters such as the UV dose of exposure received by the user's Cj body parts, weather conditions and possibly other factors due to the physical activity carried out by the user (e.g. sweating, bathing, etc.).

For this purpose, these relevant parameters can be entered and stored in the SP terminal or recorded.

Advantageously, the invention apparatus will therefore be able to include or be associated with devices, such as wearable devices, capable of detecting significant parameters for the renewal of the application of the photo protection product such as sensors of the actual exposure to solar radiation of the user's Cj body parts during the use of the apparatus and/or sensors or devices to detect or provide meteorological and/or satellite data useful for estimating the actual exposure of the Cj body parts to solar radiation and therefore the possible need to reapply protection.

According to the invention, a method of controlling the dispensed quantity of protective sunscreen is also described, by means of dispenser 1 of at least one photoprotective product Pk, equipped with dispensing media 2 that can be operated to repeatedly dispense a constant unit dose D of product Pk contained in at least one Sk-tank, including the following phases

calculate a quantity Qjk of product Pk necessary to obtain an effective desired protection factor FPEjk of one or more selected parts Cj of the user's body on the basis of the width Aj of the skin surface of the part or parts to be protected Cj and the thickness tjk of photoprotective product Pk with nominal protection factor FPk necessary to obtain the desired FPEjk value,
receive from dispenser 1 the current number Ni of dispensations made,
calculate the number of remaining dispensations Njk-Ni still to be performed to reach the total calculated number Njk, and
inform the user of the number of Njk-Ni dispensationd still to be performed.

The method of the invention may also provide that on the basis of a total number Njk of doses delivered by the user for the Cj part of the body of the product Pk extracted from the tank Sk, the effective protection factor FPEjk is calculated resulting from the calculation of the thickness tjk resulting from the application of said total number Njk of doses applied.

The present invention has been described according to preferred forms of realization, but equivalent variants can be conceived without leaving the scope of protection of the invention.

**Claims**

1. Protective sunscreen dispensing apparatus, comprising

   a dispenser (1) of at least one photo-protective product (Pk), provided with
   dispensing means (2) operable to repeatedly dispense a constant unit dose (D) of product (Pk) contained in at least one tank (Sk),
   an electronic unit (16),
   detection means (15) connected to said electronic unit (16) and configured for the detection of the unit dispensing made,
   selection means (7) connected to said electronic unit (16) and
   configured to select a part (Cj) of the body to be protected,
   a wireless interface (3a) connected to said electronic unit (16) and configured to receive from the electronic unit (16) the number (Ni) of dispensations made, detected by the detection means (15), and transmit it to a corresponding communication interface (3b) of an electronic terminal (SP) for use by a user of the apparatus,
   a computer program (APP) stored and executable in the portable device (SP) which, if executed,
   calculates an amount (Qjk) of product (Pk) necessary to obtain a desired effective protection factor (FPEjk) of one or more selected parts (Cj) of the user's body based on the skin surface width (Aj) of the part(s) to be protected (Cj) and the thickness (tjk) of photo-protective product (Pk) with nominal protection factor (FPk) necessary to obtain the desired value (FPEjk),
   calculates the number (Njk) of doses (D) to be dispensed to obtain the calculated product quantity (Qjk), equal to Qjk/D,
   receives from the dispenser (1) the current number (Ni) of dispensations made, and
   calculates the number of remaining dispensations (Njk-Ni) still to be performed to reach the calculated total number (Njk) and makes it known to the user.

2. Apparatus according to claim 1, wherein said program (APP) makes known the number of residual dispensations (Njk-Ni) by means of a screen (18) of the terminal (SP) and/or by sending by means of said interfaces (3a, 3b) the value (Njk-Ni) to the dispenser (1) for its display by means of a display (4b) of the dispenser (1).

3. Apparatus according to one of the preceding claims, wherein said dispenser (1) comprises a plurality of tanks (Sk) for containing different photo-protective products (Pk) and at least one tank selector (Sk) to be used (8), which is connected to the electronics (16) and configured to select a tank (Sk) from which to withdraw the photo-protective product (Pk) to be dispensed.

4. Apparatus according to one of the preceding claims, said selector (8) being remote-controlled by the user terminal (SP) by means of said interfaces (3a, 3b).

5. Apparatus according to one of the preceding claims, wherein said program (APP) for each part (Cj) of the human body determines the respective skin surface (Aj) based at least on biometric data of the user, already stored in the terminal (SP) or detected at the time of calculation of the number of dispensations (Njk).

6. Apparatus according to one of the preceding claims, wherein said means for selecting the body part (Cj) to be protected comprises a selector (7) of the dispenser (1) connected to said electronic unit (16) and configured to select a body part (Cj) to be protected.

7. Apparatus according to one of the preceding claims, wherein said means for selecting the body part (Cj) to be protected comprise said computer program (APP) which, if executed, selects and signals to the user a part (Cj) to be protected.

8. Apparatus according to one of the preceding claims, wherein said program (APP) is configured to calculate said number of dispensations (Njk) at least according to the user's phototype and/or information related to any portions of the skin surface not exposed to solar radiation and/or to a desired exposure time.

9. Apparatus according to one of the preceding claims, wherein said program (APP) is configured to preliminarily calculate said desired effective protection factor (FPEjk) based on the photo-protective factor (FPk) of the product (Pk) contained in the tank Sk and on further relevant factors for the photo protection of the skin for the body part (Cj) comprising subjective parameters associated with the user and/or objective parameters such as the current and expected UV radiation for the location in which the user is exposed and/or an expected exposure time of the user to solar radiation.

10. Apparatus according to one of the preceding claims, wherein said program (APP) is configured to calculate and provide the user with recommended exposure times in relation to one or more effects deriving from sun exposure.

11. Apparatus according to one of the preceding claims, wherein said dispensing means (2) comprise a mechanically operated pump (12).

12. Apparatus according to one of the preceding claims, wherein said dispensing detection means (15) comprise an on/off switch connected to the electronic unit (16).

13. Apparatus according to one of the preceding claims, comprising a graph (G) showing the parts of the human body (Cj) to be protected, said graph being displayed on the screen of the user terminal (SP) and/or on the dispenser (1) through a graphic pattern with the activation of a light indicator corresponding to the selected body part.

14. Apparatus according to one of the preceding claims, wherein said dispenser (1) comprises a container (10) comprising said tanks (Sk) closed by a removable lid (9) provided with said dispensing means (2) wherein the lid (9) comprises at least said electronic unit (16) and/or said selectors (7, 8) and/or said interface (3a) and/or said screen (4b) and/or said indicators (4a) and/or said graph (G).

15. Apparatus according to one of the preceding claims, further comprising devices for detecting the actual exposure to solar radiation of the body parts (Cj) of the user during the use of the apparatus and/or devices for detecting or providing meteorological and/or satellite data useful to estimate the actual exposure of the body parts (Cj) to solar radiation, wherein said program APP is configured to recall the user and thus the possible need to reapply a protection.

FIG.1

FIG.1a

FIG.2

FIG.3

| Europäisches Patentamt<br>European Patent Office<br>Office européen des brevets | **EUROPEAN SEARCH REPORT** | **Application Number**<br>**EP 25 17 0943** |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2020/368559 A1 (POUTIATINE ANDREW [US]) 26 November 2020 (2020-11-26)<br>* the whole document *<br>* paragraphs [0020], [0022], [0024], [0027], [0049], [0122], [0130], [0152], [0153], [0159], [0160], [0163] - [0184]; figures 1,5,6 * | 1-15 | INV.<br>A45D44/00<br>A47K5/12<br>A61M35/00 |
| Y | WO 2022/221747 A1 (JOHNSON CHRISTIAN K [US]) 20 October 2022 (2022-10-20)<br>* paragraphs [0048] - [0065], [0101], [0102]; figures 1,2 * | 1,3-5, 7-9 | |
| Y,D | US 2018/304295 A1 (O'MARA BRADLEY [US]) 25 October 2018 (2018-10-25)<br>* paragraphs [0041] - [0046]; figures 1,2 * | 1-5,7-15 | |
| A | CN 101 822 529 A (COURAGE BREWING LTD) 8 September 2010 (2010-09-08)<br>* figure 1 * | 1-15 | |
| | | | TECHNICAL FIELDS SEARCHED (IPC) |
| Y | US 2023/119899 A1 (LONDON VANESSA [US]) 20 April 2023 (2023-04-20)<br>* abstract; figures 1,2a,2B,3 *<br>* paragraphs [0004], [0007], [0024], [0027] - [0030], [0039] - [0044] * | 1-15 | A45D<br>A47K<br>A61M |

The present search report has been drawn up for all claims

| Place of search | Date of completion of the search | Examiner |
|---|---|---|
| The Hague | 27 August 2025 | Longo dit Operti, T |

EPO FORM 1503 03.82 (P04C01)

## ANNEX TO THE EUROPEAN SEARCH REPORT
## ON EUROPEAN PATENT APPLICATION NO.

EP 25 17 0943

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2025

| Patent document cited in search report | | | Publication date | Patent family member(s) | | Publication date |
|---|---|---|---|---|---|---|
| US 2020368559 | A1 | | 26-11-2020 | US 2019060678 A1 | | 28-02-2019 |
| | | | | US 2020368559 A1 | | 26-11-2020 |
| WO 2022221747 | A1 | | 20-10-2022 | AU 2022257095 A1 | | 19-10-2023 |
| | | | | CA 3216871 A1 | | 20-10-2022 |
| | | | | EP 4323123 A1 | | 21-02-2024 |
| | | | | US 2024189559 A1 | | 13-06-2024 |
| | | | | WO 2022221747 A1 | | 20-10-2022 |
| US 2018304295 | A1 | | 25-10-2018 | NONE | | |
| CN 101822529 | A | | 08-09-2010 | CN 101822529 A | | 08-09-2010 |
| | | | | EP 2226092 A1 | | 08-09-2010 |
| | | | | JP 2010201165 A | | 16-09-2010 |
| | | | | US 2010228106 A1 | | 09-09-2010 |
| US 2023119899 | A1 | | 20-04-2023 | NONE | | |

EPO FORM P0459

For more details about this annex : see Official Journal of the European Patent Office, No. 12/82

**REFERENCES CITED IN THE DESCRIPTION**

**Patent documents cited in the description**

- US 9551611 B